# EUROPEAN PATENT APPLICATION

(11) **EP 4 428 144 A1**
(43) Date of publication of application: **11.09.2024**
(21) Application number: 22890288.8
(22) Date of filing: 28.10.2022
(51) Int. Cl.: C07K 7/06, C12N 5/077, A61K 38/00, A61P 19/08, A61P 29/00, A23L 33/18

(54) **PEPTIDE HAVING PHYSIOLOGICAL ACTIVITY AND USE THEREOF**

(30) Priority: 04.11.2021 KR 20210150631
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); YEON, Jeong Tae, Anyang-si, Gyeonggi-do 14119 (KR); LEE, Eun Ju, Anyang-si, Gyeonggi-do 14119 (KR); CHO, Hyun Joo, Anyang-si, Gyeonggi-do 14119 (KR)
(74) Representative: Regimbeau
(86) International application number: PCT/KR2022/016663
(87) International publication number: WO 2023/080553

(57) **Abstract**

The present invention relates to a novel peptide having various physiological activities and use thereof. The novel peptide according to the present invention induces an increase in joint components in chondrocytes, increases the cartilage differentiation of mesenchymal stem cells and the production of extracellular matrix, suppresses an increase in inflammatory cytokines or inflammatory proteins, and can also suppress the differentiation and formation of osteoclasts, and thus can be effectively used as an active ingredient of a pharmaceutical composition for regenerating cartilage or preventing or treating an inflammatory disease or osteoporosis, or a health functional food for ameliorating the symptoms.

## Description

### TECHNICAL FIELD

The present invention relates to a peptide having various physiological activities and a use thereof.

### BACKGROUND ART

In general, once cartilage tissue that forms the joints of vertebrates is damaged, the cartilage tissue is not normally regenerated in vivo because it does not have blood vessels, nerves, or lymphatic tissue. When the cartilage tissue of the joints is damaged, severe pain and limitations in daily activities are caused, and when it becomes chronic, fatal degenerative arthritis is caused, which interferes with normal life or professional activity. Therefore, damaged articular cartilage has significantly limited self-recovery and regeneration. Although various medical methods are being pursued, these methods are still insufficient to restore hyaline cartilage, which is the joint cartilage, to its natural state.

As a surgical method for clinically regenerating cartilage, a method of inducing differentiation of stem cells into chondrocytes, a method of transplanting autologous or allogeneic cartilage tissue to a cartilage defect site using osteochondral transplantation, a method of inducing regeneration of marrow cells flowing out through holes into cartilage tissue by carefully scraping and removing damaged cartilage and drilling holes at predetermined intervals when the subchondral bone is exposed using microfracture, a method of inducing cartilage regeneration by transplanting chondrocytes into a cartilage defect site using chondrocyte transplantation, a method of using autologous chondrocyte transplantation to treat damaged articular cartilage, or the like, has been used.

However, when using the above methods, surgery is required each time to collect chondrocytes or to transplant chondrocytes cultured in vitro back to a damaged articular cartilage site, ultimately resulting in pain, aftereffects, financial burden on a patient due to two rounds of surgery, and a surgical process is also complicated. In addition, since most of the collected chondrocytes are obtained from adults who have already completed their growth, proliferation and growth of the collected cells are not active, and thus it takes a considerable period of time to obtain the number of cells required for transplantation when culturing the cells in vitro, in a case where cells lose the ability to differentiate and do not proliferate at all, a treatment itself cannot be achieved, and a cell's phenotype often changes because chondrocytes are cultured in vitro. Therefore, there is a need to develop a technology capable of more effectively regenerating cartilage tissue.

Meanwhile, inflammation is a type of defensive response of biological tissue that occurs when tissue or cells are damaged or injured, or infected by an external source of infection (viruses, bacteria, fungi, allergens, or the like), and refers to a series of complex conditions caused by immune cells involved in various immune responses that gather around damaged or infected sites and inflammatory factors secreted by the immune cells.

Currently known therapeutic agents for inflammatory diseases include dexamethasone and cortisone using adrenocortical hormone components, but although these therapeutic agents are active as therapeutic agents, they are highly toxic and may cause side effects such as edema. In addition, since these therapeutic agents do not selectively act on the cause of inflammation, it has been reported that severe levels of immunosuppression may occur, which may cause problems. As described above, the treatment of inflammatory diseases using a drug containing a steroid ingredient is accompanied by side effects and problems, and therefore, there is an urgent need to develop a therapeutic agent for inflammatory diseases that may have a stable inflammation inhibition treatment effect without side effects using a non-steroidal drug.

In addition, various types of compounds that inhibit bone resorption or promote bone formation are currently disclosed as therapeutic agents for osteoporosis, but these conventional therapeutic agents often have toxicity and side effects, and thus, the development of a new substance to replace them is required.

### DISCLOSURE OF THE INVENTION

### TECHNICAL PROBLEM

An object of the present invention is to provide a peptide having various physiological activities.

In addition, another object of the present invention is to provide a pharmaceutical composition for regenerating cartilage or preventing or treating an inflammatory disease or osteoporosis, the pharmaceutical composition containing the peptide as an active ingredient.

In addition, still another object of the present invention is to provide a health functional food for regenerating cartilage or preventing or ameliorating an inflammatory disease or osteoporosis, the health functional food containing the peptide as an active ingredient.

In addition, still another object of the present invention is to provide a method for treating, preventing, or ameliorating osteoporosis, the method including administering a therapeutically effective amount of the peptide to a subject.

Still another object of the present invention is to provide a use of the peptide for regenerating cartilage or treating, preventing, or ameliorating an inflammatory disease or osteoporosis.

### TECHNICAL SOLUTION

In order to achieve the above objects, an aspect of the present invention provides a peptide containing an amino acid sequence of SEQ ID NO: 1.

In an embodiment, the peptide may have various physiological activities such as cartilage regenerative activity, anti-inflammatory activity, and osteoporosis inhibitory activity.

In an embodiment, the peptide of the present invention may promote differentiation of stem cells such as cord blood-derived stem cells, peripheral blood-derived stem cells, bone marrow-derived stem cells, and mesenchymal stem cells, and preferably mesenchymal stem cells, into chondrocytes.

In an embodiment, the peptide of the present invention may increase synthesis of an extracellular matrix (ECM) in chondrocytes. In addition, the peptide of the present invention may increase expression of genes associated with ECM production such as collagen type II (COL2A1), aggrecan (ACAN), and proteoglycan core protein (PCP) in chondrocytes.

In another embodiment, the peptide of the present invention may inhibit expression of inflammatory cytokines. Examples of the inflammatory cytokines include TNFα, IL-1β, COX2, IL-6, IL-17, and IFNγ, but are not limited thereto.

In still another embodiment, the peptide of the present invention may inhibit differentiation of macrophages into osteoclasts.

Another aspect of the present invention provides a pharmaceutical composition for regenerating cartilage or preventing or treating an inflammatory disease or osteoporosis, the pharmaceutical composition containing the peptide containing the amino acid sequence of SEQ ID NO: 1 as an active ingredient.

Still another aspect of the present invention provides a health functional food for regenerating cartilage or preventing or ameliorating an inflammatory disease or osteoporosis, the health functional food containing the peptide containing the amino acid sequence of SEQ ID NO: 1 as an active ingredient.

Still another aspect of the present invention provides a method for treating, preventing, or ameliorating osteoporosis in a subject, the method including administering a therapeutically effective amount of the peptide containing the amino acid sequence of SEQ ID NO: 1 to a subject, for example, an osteoporosis patient.

Still another aspect of the present invention provides a method for treating, preventing, or ameliorating an inflammatory disease in a subject, the method including administering a therapeutically effective amount of the peptide containing the amino acid sequence of SEQ ID NO: 1 to a subject, for example, a patient suffering from an inflammatory disease.

Still another aspect of the present invention provides a method for regenerating cartilage in a subject, the method including administering a therapeutically effective amount of the peptide containing the amino acid sequence of SEQ ID NO: 1 to a subject, for example, a patient in need of cartilage regeneration.

Still another aspect of the present invention provides a use of the peptide containing the amino acid sequence of SEQ ID NO: 1 for treating, preventing, or ameliorating osteoporosis or an inflammatory disease.

Still another aspect of the present invention provides a use of the peptide containing the amino acid sequence of SEQ ID NO: 1 for regenerating cartilage.

### ADVANTAGEOUS EFFECTS

The peptide provided in the present invention may induce an increase in constituent components of joints in chondrocytes, may increase chondrogenic differentiation of mesenchymal stem cells and production of an extracellular matrix (ECM), may inhibit an increase in inflammatory cytokines or inflammatory proteins, and may also inhibit differentiation and formation of osteoclasts. Therefore, the peptide may be effectively used as an active ingredient in a pharmaceutical composition for regenerating cartilage or preventing or treating an inflammatory disease or osteoporosis, or a health functional food for alleviating such symptoms.

However, the effects of the present invention are not limited to the effects mentioned above, and other effects that are not mentioned may be obviously understood by those skilled in the art from the following description.

### BRIEF DESCRIPTION OF THE DRAWINGS

FIGS. 1A and 1B illustrate molecular weight analysis results showing synthesis of a peptide of the present invention.
FIG. 2 is an alcian blue staining photograph showing an effect of the peptide of the present invention on ECM synthesis in chondrocytes.
FIG. 3 illustrates an electrophoresis photograph and a graph showing the effect of the peptide of the present invention on expression of ECM-related genes in chondrocytes.
FIG. 4 is an alcian blue staining photograph showing the effect of the peptide of the present invention on synthesis of ECM molecules in three-dimensional culture of chondrocytes, in which NON represents a group that is not treated with the peptide of the present invention.
FIG. 5 is an alcian blue staining photograph showing the effect of the peptide of the present invention on differentiation of mesenchymal stem cells (MSCs) into chondrocytes and ECM production.
FIGS. 6A to 6C are electrophoresis photographs showing the effect of the peptide of the present invention on production of SOX9 protein, which is a regulator of ECM synthesis, in mesenchymal stem cells, in which FIGS. 6A, 6B, and 6C illustrate the results after 3, 7, and 14 days of treatment with the peptide of the present invention, respectively. In FIGS. 6A to 6C, Non represents a group that is not treated with the peptide of the present invention, and CM represents a positive control.
FIGS. 7A to 7C are electrophoresis photographs showing the effect of the peptide of the present invention on the expression of ECM-related genes in mesenchymal stem cells, in which FIGS. 7A, 7B, and 7C illustrate the results after 3, 7, and 14 days of treatment with the peptide of the present invention, respectively. In FIGS. 7A to 7C, Non represents a group that is not treated with the peptide of the present invention.
FIG. 8 illustrates an electrophoresis photograph and a graph showing the effect of the peptide of the present invention on expression of inflammatory cytokines in macrophages in which an inflammatory response is induced, in which CON represents a group that is not treated with the peptide of the present invention.
FIG. 9 illustrates an electrophoresis photograph and a graph showing the effect of the peptide of the present invention on production of COX2, which is an inflammatory protein, in macrophages in which an inflammatory response is induced, in which CON represents a group that is not treated with the peptide of the present invention.

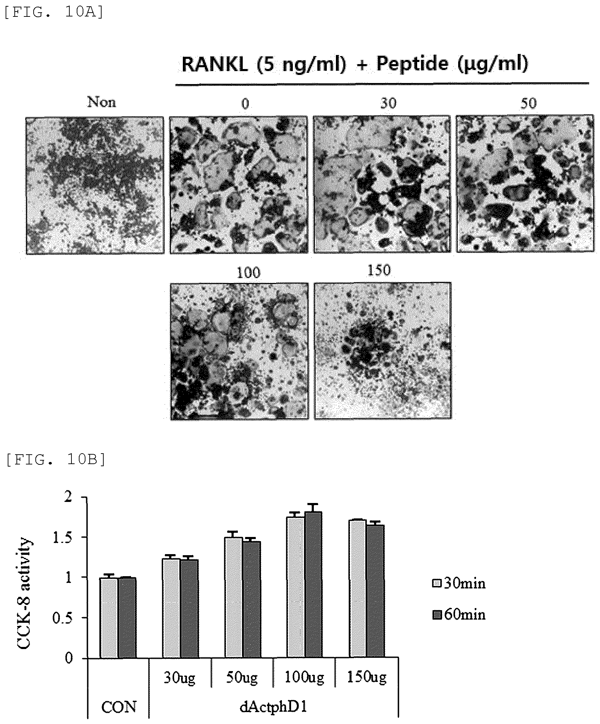

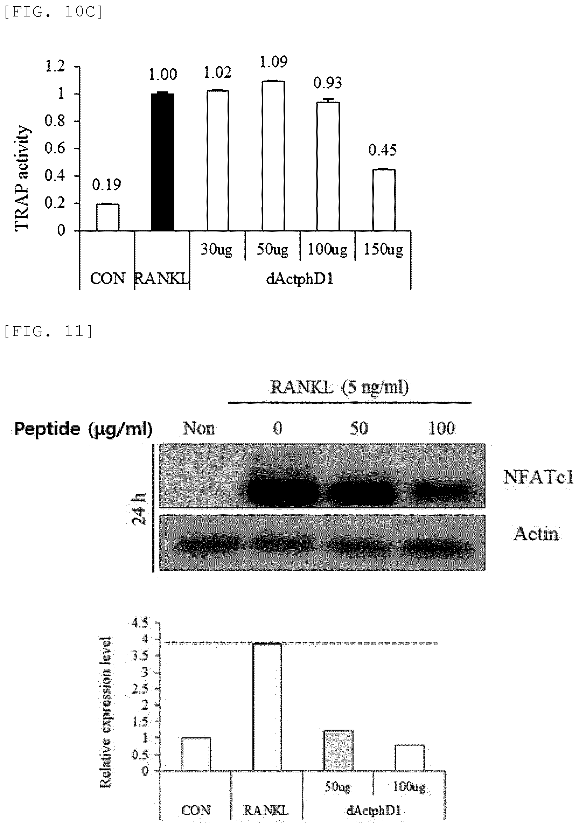

FIGS. 10A to 10C are staining photographs and graphs showing the effect of the peptide of the present invention on differentiation of macrophages into osteoclasts, in which FIG. 10A is a staining photograph showing that differentiation into osteoclasts induced by RANKL is inhibited by the peptide of the present invention, FIG. 10B is a graph showing that the peptide of the present invention does not show toxicity in mouse macrophages, and FIG. 10C is a graph showing that the peptide of the present invention inhibits the activity of TRAP, which is a marker of osteoclasts, in a concentration-dependent manner. In FIGS. 10A to 10C, Non represents a group that is not treated with the peptide of the present invention.
FIG. 11 is a staining photograph showing the effect of the peptide of the present invention on differentiation of macrophages into osteoclasts, in which CON represents a group that is not treated with the peptide of the present invention.
FIG. 12 is an immunofluorescence staining photograph showing the effect of the peptide of the present invention on formation of an actin ring, which is essential for differentiation of macrophages into osteoclasts.

### MODE FOR CARRYING OUT THE INVENTION

Hereinafter, the present invention will be described in detail.

### 1. Peptide of the present invention

The present invention provides a peptide having useful physiological activities such as cartilage regenerative activity, anti-inflammatory activity, and osteoporosis inhibitory activity.

The peptide refers to a polymer consisting of two or more amino acids linked by a peptide bond. When the size of the peptide itself is too large, it is not effectively introduced into target tissue or cells or disappears in the body in a short period of time due to a short half-life, and therefore, the peptide of the present invention consists of 20 or less, for example, 15 or less or 12 or less amino acids.

The peptide of the present invention may contain an amino acid sequence of SEQ ID NO: 1 or may consist of the amino acid sequence. In an embodiment of the present invention, as for the peptide of the present invention, all variants or fragments of amino acids having a different sequence from SEQ ID NO: 1 in which amino acid residues constituting the amino acid sequence of SEQ ID NO: 1 are modified by deletion, insertion, substitution, or a combination thereof may also be included in the peptide of the present invention within a range that does not affect useful physiological activities such as cartilage regenerative activity, anti-inflammatory activity, and osteoporosis inhibitory activity. Amino acid exchange at the peptide level that does not totally change the physiological activities of the peptide, such as cartilage regenerative activity, anti-inflammatory activity, and osteoporosis inhibitory activity is known in the art. In some cases, the peptide may be modified by phosphorylation, sulfation, acrylation, glycosylation, methylation, farnesylation, or the like. Therefore, the present invention includes a peptide containing an amino acid sequence that is substantially identical to that of a peptide containing the amino acid sequence of SEQ ID NO: 1 or consisting of the amino acid sequence, or a variant thereof or an active fragment thereof. The substantially identical protein refers to an amino acid sequence having 75% or more, for example, 80% or more, 90% or more, 95% or more, 98% or more, or 99% or more sequence homology with the amino acid sequence of SEQ ID NO: 1. In addition, the peptide of the present invention may further contain a targeting sequence, a tag, a labeled residue, or an amino acid sequence prepared for a specific purpose to increase half-life or stability of the peptide.

In addition, the peptide of the present invention may be obtained by various methods commonly known in the art. In an embodiment of the present invention, the peptide of the present invention may be produced using polynucleotide recombination and protein expression systems, or may be produced by an in vitro synthesis method through chemical synthesis such as peptide synthesis, a cell-free protein synthesis method, or the like.

In addition, in order to obtain better chemical stability, enhanced pharmacological properties (half-life, absorbency, potency, efficacy, and the like), modified specificity (for example, a broad biological activity spectrum), and reduced antigenicity, a protecting group may bind to an N-terminus or C-terminus of the peptide. For example, the protecting group may be an acetyl group, a fluorenylmethoxycarbonyl group, a formyl group, a palmitoyl group, a myristyl group, a stearyl group, or a polyethylene glycol (PEG) group, and may include, but is not limited to, any ingredient capable of enhancing modification of the peptide, particularly, stability of the peptide. The "stability" is used to include not only in vivo stability for protecting the peptide of the present invention from attack of a protein cleavage enzyme in vivo, but also storage stability (for example, room-temperature storage stability).

In the present invention, "cartilage regenerative activity" is used to include, but is not limited to, a series of in vivo or in vitro activities required for formation of cartilage. In an embodiment of the present invention, the cartilage regenerative activity may include an activity that promotes the synthesis of components constituting cartilage, such as an ECM, in chondrocytes. In another embodiment of the present invention, the cartilage regenerative activity may include an activity that increases expression of an ECM regulator in chondrocytes. In another embodiment of the present invention, the cartilage regenerative activity may include an activity that promotes differentiation of stem cells such as cord blood-derived stem cells, peripheral blood-derived stem cells, bone marrow-derived stem cells, and mesenchymal stem cells, and preferably mesenchymal stem cells, into chondrocytes. In another embodiment of the present invention, the cartilage regenerative activity may include an activity that promotes production of an ECM and an ECM regulatory factor in chondrocytes or stem cells, and preferably mesenchymal stem cells, or promotes expression of genes related thereto.

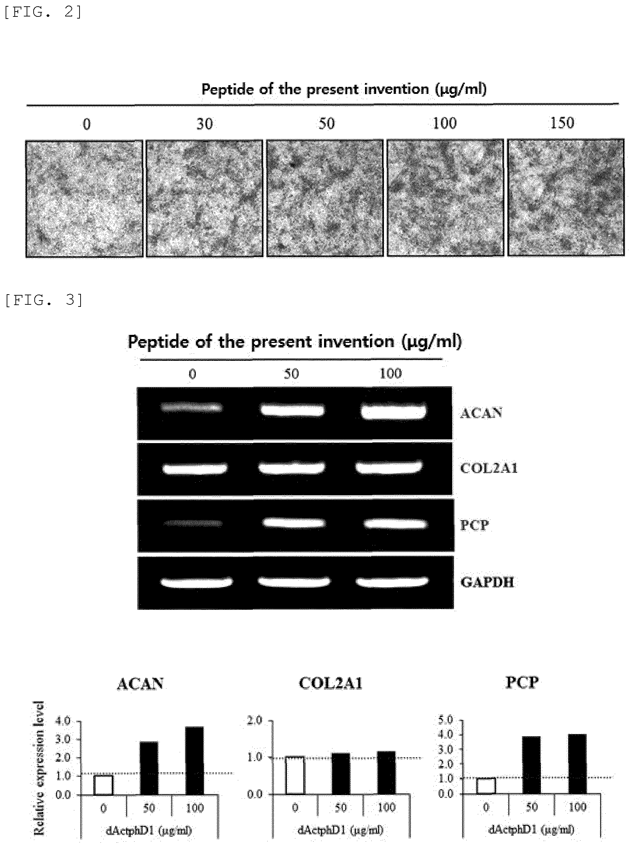

In order to confirm the cartilage regeneration effect of the peptide of the present invention, in a preferred embodiment of the present invention, as a result of confirming ECM formation by treating human chondrocytes with the peptide containing the amino acid sequence of SEQ ID NO: 1, it was confirmed that ECM production in chondrocytes was promoted by the treatment with the peptide of the present invention (see FIG. 2).

In addition, in another embodiment, as a result of confirming expression levels of genes involved in ECM formation by treating human chondrocytes with the peptide containing the amino acid sequence of SEQ ID NO: 1, it was confirmed that the expression of COL2A1, COMP, and PCP genes present in chondrocytes was promoted by the treatment with the peptide of the present invention (see FIG. 3).

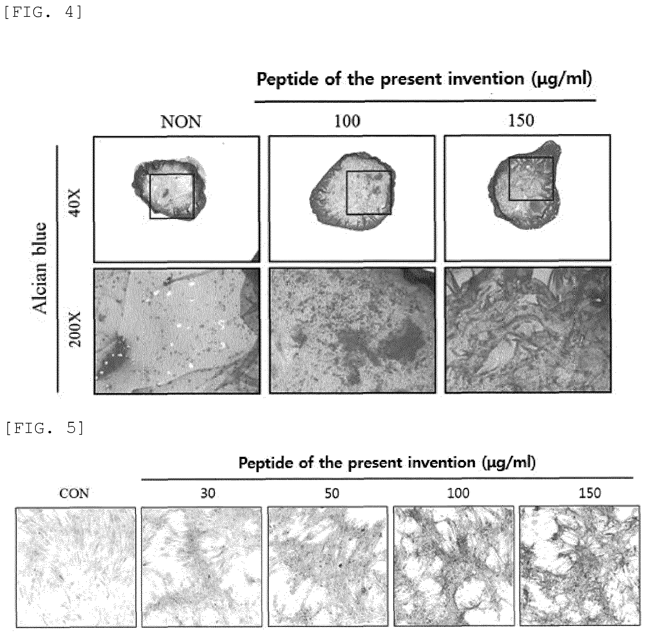

In addition, in still another embodiment of the present invention, as a result of confirming ECM formation through treatment with the peptide containing the amino acid sequence of SEQ ID NO: 1 in three-dimensional culture of human chondrocytes, it was confirmed that production of signaling proteins involved in ECM formation in chondrocytes was increased by the treatment with the peptide of the present invention (see FIG. 4).

In addition, in still another embodiment of the present invention, as a result of confirming differentiation of mesenchymal stem cells into chondrocytes and ECM production, it was confirmed that the differentiation of mesenchymal stem cells into chondrocytes and ECM production were promoted by the treatment with the peptide of the present invention (see FIG. 5).

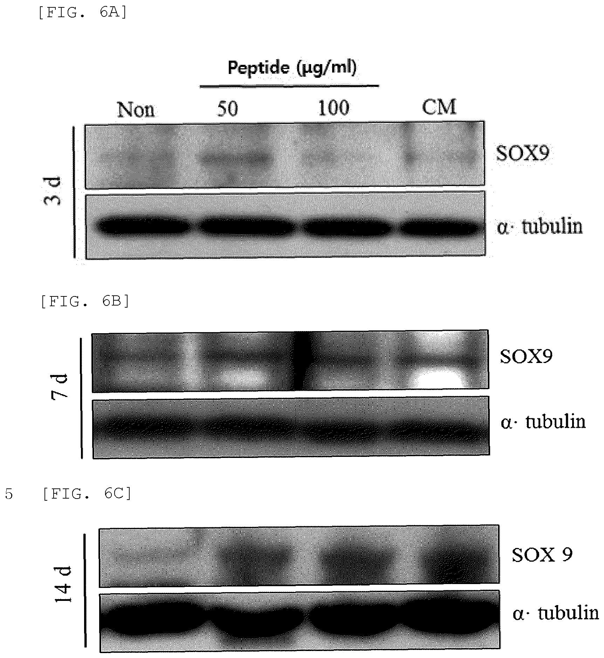

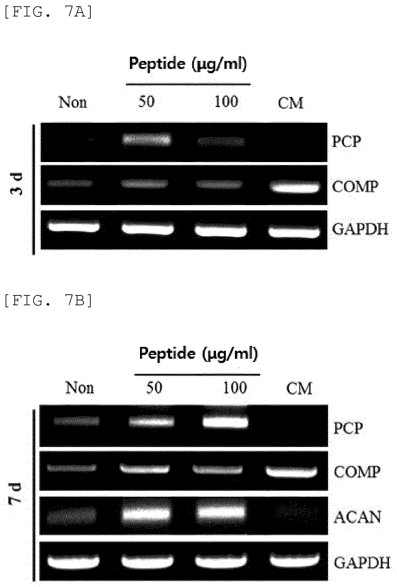

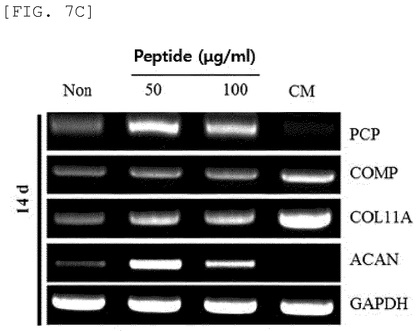

In addition, in still another embodiment of the present invention, as a result of confirming ECM production and expression levels of genes involved in ECM formation by treating mesenchymal stem cells with the peptide containing the amino acid sequence of SEQ ID NO: 1, it was confirmed that production of an ECM present in mesenchymal stem cells and the expression of genes and proteins that regulate ECM production were promoted by the treatment with the peptide of the present invention (see FIGS. 6A to 6C and FIGS. 7A to 7C).

Meanwhile, the peptide of the present invention may have physiological activities other than cartilage regeneration, such as anti-inflammatory activity.

In the present invention, the "anti-inflammatory activity" is used to include, but is not limited to, an activity that inhibits inflammation, and the inflammation is a type of defensive response of biological tissue to any stimulus and refers to a pathological condition of an abscess that is formed when tissue or cells are damaged or infected with various sources of infection such as bacteria, fungi, viruses, and allergens derived from the outside. In addition, the inflammatory response is a complex physiological response such as enzyme activation, secretion of inflammatory mediators, body fluid infiltration, cell migration, or tissue destruction that occurs in local blood vessels and body fluids in association with inflammatory mediators and immune cells, and an external symptom such as erythema, edema, fever, or pain. The peptide of the present invention has an effect of reducing and alleviating a series of pathological conditions and symptoms as described above because it has the activity of inhibiting inflammation.

In addition, the peptide of the present invention may inhibit expression of inflammatory cytokines or inflammatory proteins. In the inflammatory response, when a wound occurs or an external pathogen that invades the wound enters the body, white blood cells responsible for the early stage immune response gather around the wound or pathogen, and inflammation-related cytokines or proteins are expressed and secreted to induce an inflammatory response, and thus, anti-inflammatory activity may be exhibited by inhibiting the expression of inflammatory cytokines or inflammatory proteins. In addition, the anti-inflammatory activity and inflammation inhibitory effect of the peptide of the present invention may be confirmed by confirming the expression levels of the inflammatory cytokines or proteins.

The inflammatory cytokines may be one or more selected from the group consisting of TNFα, IL-6, IL-17, IL-1β, and IFNγ. The TNFα is an abbreviation for "Tumor Necrosis Factor α", is a cytokine produced and secreted by macrophages and various cells activated during the immune response to bacterial infection or tumor disease, is known to be a major mediator of the inflammatory response, and plays an important role in inflammatory diseases such as rheumatoid arthritis (RA), psoriatic arthritis, Crohn's disease, psoriasis, and ankylosing spondylitis (AS). The IL-6 (interleukin 6) is a cytokine produced by macrophages and various lymphocytes, plays a role in promoting an inflammatory response, and is known to cause inflammatory diseases when produced excessively. The IL-17 (interleukin 17) is also a pro-inflammatory cytokine, is produced from Th17 cells, and plays a role in inducing or mediating an inflammatory response. The IFNγ (interferon γ) may be produced in T lymphocytes and macrophages, is secreted in response to infection by viruses or bacteria invading from the outside, and is known to play a role in relation to autoimmune or autoinflammatory diseases. Therefore, the peptide of the present invention has the effect of inhibiting inflammation through its activity of inhibiting the expression of inflammatory cytokines as described above and inhibiting the secretion of expressed cytokines.

In addition, the peptide of the present invention may inhibit expression of Cox2. The Cox2 (cyclooxygenase 2) is an enzyme that stimulates and participates in a biosynthetic process of prostaglandins, may be expressed under regulation of NF-κB, and regulates an inflammatory response. Since Cox2 is a protein that is rarely expressed under normal conditions, but is rapidly expressed by stimulation of cytokines, inflammatory factors, endotoxin, or the like, the anti-inflammatory activity of the peptide of the present invention may be confirmed by confirming the expression level of Cox2 gene or the amount of Cox2 protein, and the peptide of the present invention has the effect of inhibiting inflammation by inhibiting the expression of Cox2.

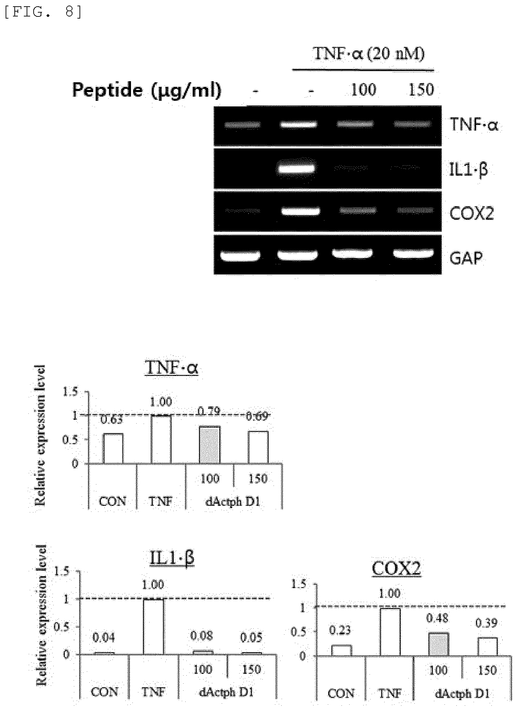

In order to confirm the inflammation inhibitory effect of the peptide of the present invention, in a preferred embodiment of the present invention, as a result of confirming the amount of Cox2 protein by treating mouse macrophages with inflammation-inducing cytokines together with the peptide containing the amino acid sequence of SEQ ID NO: 1, it was confirmed that the amount of Cox2 protein decreased according to the treatment with the peptide of the present invention even though the inflammatory response of cells was induced by the treated inflammation-inducing cytokines (see FIG. 8).

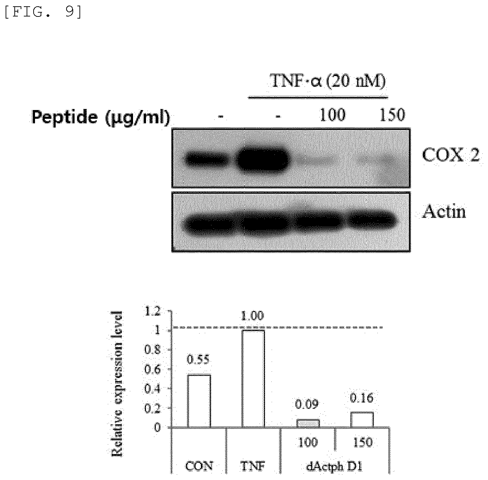

In addition, in order to confirm the effect of reducing the expression levels of inflammation-related genes according to the treatment with the peptide of the present invention, in a preferred embodiment of the present invention, as a result of confirming the amount of mRNA for TNFα, IL-1β, and Cox2 genes by treating mouse macrophages with inflammation-inducing cytokines together with the peptide containing the amino acid sequence of SEQ ID NO: 1, it was confirmed that the expression levels of the inflammation-inducing cytokines or enzyme genes related thereto decreased according to the treatment with the peptide of the present invention even though the inflammatory response of cells was induced by the treated inflammation-inducing cytokines (see FIG. 9).

Therefore, it is clear that the peptide of the present invention has anti-inflammatory activity that may reduce and ameliorate inflammatory responses because it reduces the expression or secretion of inflammatory cytokines such as TNFα, IL-6, IL-17, IL-1β, and IFNγ, which may promote inflammatory responses, and inhibits the expression of inflammation-related factors such as Cox2. Accordingly, the peptide of the present invention may be effectively used as an active ingredient in a composition for preventing, treating, or ameliorating an inflammatory disease caused by inflammation or accompanied by an inflammatory response.

In addition, the peptide of the present invention may have physiological activities other than cartilage regeneration, such as osteoclast inhibitory activity.

In an embodiment of the present invention, as a result of confirming differentiation into osteoclasts and the activity of proteins related to osteoclast differentiation by treating mouse macrophages with a receptor activator of nuclear factor Kappa-B ligand (RANKL) that induces differentiation into osteoclasts together with the peptide containing the amino acid sequence of SEQ ID NO: 1, it was confirmed that the differentiation into osteoclasts and the expression of proteins related thereto were reduced, and the formation of actin rings, which are essential for osteoclast differentiation, was inhibited according to the treatment with the peptide of the present invention (see FIGS. 10A to 10C and FIGS. 11 and 12).

The increase in activity of osteoclasts is closely related to osteoporosis, and therefore, the peptide of the present invention may be effectively used in the prevention or treatment of osteoporosis by inhibiting the activity of osteoclasts.

### 2. Pharmaceutical composition containing peptide of the present invention

Another aspect of the present invention provides a pharmaceutical composition for regenerating cartilage containing the peptide containing the amino acid sequence of SEQ ID NO: 1 as an active ingredient.

Still another aspect of the present invention provides a pharmaceutical composition for preventing or treating an inflammatory disease containing the peptide containing the amino acid sequence of SEQ ID NO: 1 as an active ingredient.

Still another aspect of the present invention provides a pharmaceutical composition for preventing or treating osteoporosis containing the peptide containing the amino acid sequence of SEQ ID NO: 1 as an active ingredient.

Since the peptide containing the amino acid sequence of SEQ ID NO: 1 is the same as the peptide described in the section of "1. Peptide of the present invention", for detailed description thereof, the section of "1. Peptide of the present invention" is used, and hereinafter, only unique features of the pharmaceutical composition will be described.

According to an embodiment of the present invention, since the peptide of the present invention has the effect of promoting cartilage regeneration, a pharmaceutical composition containing the peptide as an active ingredient may be used for regenerating cartilage.

According to another embodiment of the present invention, since the peptide of the present invention has the effect of inhibiting the expression or secretion of inflammation-related cytokines or inflammation-related factors, a pharmaceutical composition containing the peptide as an active ingredient may be used for preventing or treating an inflammatory disease.

According to still another embodiment of the present invention, since the peptide of the present invention has the effect of inhibiting the differentiation and activity of osteoclasts, a pharmaceutical composition containing the peptide as an active ingredient may be used for preventing or treating osteoporosis.

In the present invention, the inflammatory disease may refer to a pathological condition that causes inflammation caused by neutrophil chemotaxis among white blood cells, and may include, but is not limited to, any disease caused by an inflammatory response or accompanied by an inflammatory response. Examples of the inflammatory disease include, but are not limited to, rhinitis, bronchitis, periodontitis, pancreatitis, gastritis, gastric ulcer, an inflammatory skin disease, atopic dermatitis, encephalitis, sepsis, inflammatory enteritis, a chronic obstructive pulmonary disease, septic shock, pulmonary fibrosis, undifferentiated spondyloarthropathy, undifferentiated arthropathy, arthritis, inflammatory osteolysis, a chronic inflammatory disease caused by a chronic virus or bacterial infection, colitis, an inflammatory bowel disease, type 1 diabetes, rheumatoid arthritis, reactive arthritis, osteoarthritis, psoriasis, scleroderma, osteoporosis, atherosclerosis, myocarditis, endocarditis, pericarditis, cystic fibrosis, Hashimoto's thyroiditis, Graves' disease, leprosy, syphilis, Lyme disease, borreliosis, neurological borrelia, tuberculosis, sarcoidosis, lupus, discoid lupus, chilblain lupus, lupus nephritis, systemic lupus erythematosus, macular degeneration, uveitis, irritable bowel syndrome, Crohn's disease, Sjogren's syndrome, fibromyalgia, chronic fatigue syndrome, chronic fatigue immune dysfunction syndrome, myalgic encephalomyelitis, amyotrophic lateral sclerosis, Parkinson's disease, and multiple sclerosis.

The pharmaceutical composition of the present invention may be used for promoting regeneration of cartilage, preventing an inflammatory disease from occurring by inhibiting the expression or secretion of factors that cause inflammatory responses, preventing osteoporosis from occurring by inhibiting osteoclast differentiation, or preventing the worsening of the condition and treating the diseases by inhibiting inflammatory responses that occur additionally in damaged or scarred cells in patients with inflammatory diseases or the differentiation into osteoclasts in patients with osteoporosis.

Meanwhile, a pharmaceutical composition containing the peptide of the present invention as an active ingredient may be formulated using a pharmaceutically acceptable carrier and/or an excipient by a method that may be easily carried out by those skilled in the art to which the present invention pertains to be prepared in a unit dose form or to be contained in a multi-dose container. In this case, the formulation may be a solution in oil or an aqueous medium, a suspension, an emulsifying solution, an extract, a powder, a granule, a tablet, a capsule, or a gel (for example, hydrogel), and may further include a dispersant or a stabilizing agent.

In addition, the peptide contained in the pharmaceutical composition may be carried in a pharmaceutically acceptable carrier such as a colloidal suspension, a powder, saline, lipids, liposomes, microspheres, or nano-spherical particles. These peptides may form or be related to a complex with a carrying means and may be carried in vivo by using a carrying system known in the art, such as lipids, liposomes, microparticles, gold, nanoparticles, a polymer, a condensation reagent, polysaccharides, a polyamino acid, a dendrimer, saponin, an adsorption enhancing substance, or a fatty acid.

Besides, the pharmaceutically acceptable carrier may include, but is not limited to, lactose, dextrose, sucrose, sorbitol, mannitol, starch, acacia, rubber, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil, which are generally used in formulation. In addition, the pharmaceutical composition may further include a lubricant, a wetting agent, a sweetening agent, a flavoring agent, an emulsifying agent, a suspending agent, a preservative, and the like, in addition to the above ingredients.

The pharmaceutical composition according to the present invention may be administered orally or parenterally during clinical administration, and may be used in the form of a general pharmaceutical formulation. That is, the pharmaceutical composition of the present invention may be administered in various oral and parenteral dosage forms at the time of actual clinical administration, and when formulated, a diluent or an excipient such as a filler, an extender, a binder, a wetting agent, a disintegrant, or a surfactant that is commonly used in the formulation is used. A solid formulation for oral administration includes a tablet, a pill, a powder, a granule, a capsule, and the like, and such a solid formulation is prepared by mixing at least one excipient, for example, starch, calcium carbonate, sucrose, lactose, gelatin, or the like, with an herbal medicine extract or a fermented herbal medicine. Moreover, in addition to the simple excipient, lubricants such as magnesium stearate and talc are used. A liquid formulation for oral administration includes a suspending agent, an oral liquid, an emulsion, a syrup, and the like, and various excipients such as a wetting agent, a sweetening agent, a fragrance, and a preservative, in addition to water and liquid paraffin, which are commonly used simple diluents, may be included. A formulation for parenteral administration includes a sterilized aqueous solution, a non-aqueous solvent, a suspending agent, an emulsion, a freeze-dried formulation, and a suppository. As the non-aqueous solvent and the suspending solvent, propylene glycol, polyethylene glycol, vegetable oil such as olive oil, an injectable ester such as ethyl oleate, and the like may be used. As a base for the suppository, Witepsol, Macrogol, Tween 61, cacao butter, laurin butter, glycerol, gelatin, and the like may be used.

The pharmaceutical composition of the present invention is administered in a pharmaceutically effective amount. In the present invention, the "pharmaceutically effective amount" refers to an amount sufficient to treat a disease with a reasonable benefit/risk ratio applicable for medical treatment. An effective dose level may be determined according to factors including the type of disease and severity of a patient, activity of a drug, sensitivity to a drug, an administration time, a route of administration, an emission rate, a duration of treatment, and drugs used simultaneously, and other factors well-known in the medical field. The pharmaceutical composition according to the present invention may be administered as a separate therapeutic agent or in combination with another therapeutic agent for inflammatory diseases, may be administered simultaneously, separately, or sequentially with a conventional therapeutic agent, or may be administered singly or multiply. Considering all the factors, it is important to administer the pharmaceutical composition in an amount capable of obtaining a maximum effect with a minimal amount without side-effects, and the amount of the pharmaceutical composition to be administered may be easily determined by those skilled in the art.

Specifically, the effective amount of the pharmaceutical composition of the present invention may be changed according to the patient's age, gender, condition, weight, absorbency of the active ingredient in vivo, inactivation rate, excretion rate, disease type, and drug used in combination, and may be increased or decreased according to the route of administration, the severity of obesity, the gender, the weight, the age, or the like. As an example, the peptide of the present invention may be administered in an amount of about 0.0001 µg to 500 mg, for example, 0.01 µg to 100 mg per 1 kg of the patient's weight per day. In addition, the peptide of the present invention may be administered in divided doses several times a day, for example, two or three times a day, at regular time intervals, depending on the judgment of a doctor or a pharmacist.

### 3. Health functional food containing peptide of the present invention

Still another aspect of the present invention provides a health functional food for regenerating cartilage containing the peptide containing the amino acid sequence of SEQ ID NO: 1 as an active ingredient.

Still another aspect of the present invention provides a health functional food for preventing or ameliorating an inflammatory disease containing the peptide containing the amino acid sequence of SEQ ID NO: 1 as an active ingredient.

Still another aspect of the present invention provides a health functional food for preventing or ameliorating osteoporosis containing the peptide containing the amino acid sequence of SEQ ID NO: 1 as an active ingredient.

Since the peptide containing the amino acid sequence of SEQ ID NO: 1 is the same as the peptide described in the section of "1. Peptide of the present invention", for detailed description thereof, the section of "1. Peptide of the present invention" is used, and hereinafter, only unique features of the health functional food will be described.

As in the pharmaceutical composition, the health functional food containing the peptide of the present invention as an active ingredient may be effectively used for regenerating cartilage or preventing or ameliorating of an inflammatory disease or osteoporosis.

The health functional food may be used simultaneously or separately with a drug for treatment before or after the onset of the corresponding disease in order to prevent or ameliorate the disease.

In the health functional food of the present invention, the active ingredient may be added directly to the food or used together with other foods or food ingredients, and may be used appropriately according to conventional methods. A mixing amount of the active ingredient may be appropriately determined depending on the purpose of use (prevention or amelioration). In general, when producing food or beverages, the composition of the present invention may be added in an amount of preferably 15 wt% or less and more preferably 10 wt% or less with respect to the raw materials. However, in the case of long-term intake for the purpose of health and hygiene or health control, the amount may be below the above range.

The health functional food may contain other ingredients as essential ingredients without particular limitation, in addition to containing the active ingredient. For example, like ordinary beverages, various flavoring agents, natural carbohydrate, or the like may be contained as an additional ingredient. Examples of the natural carbohydrate include common sugars, for example, a monosaccharide such as glucose and fructose, a disaccharide such as maltose and sucrose, and a polysaccharide such as dextrin and cyclodextrin; and sugar alcohols such as xylitol, sorbitol, and erythritol. As flavoring agents other than the flavoring agents described above, a natural flavoring agent (thaumatin or a stevia extract (for example, rebaudioside A, glycyrrhizin, or the like)) and a synthetic flavoring agent (saccharin, aspartame, or the like) may be advantageously used. A proportion of the natural carbohydrate may be appropriately determined by the choice of those skilled in the art.

In addition to the above, the health functional food of the present invention may contain various nutrients, a vitamin, a mineral (electrolyte), a flavor such as a synthetic flavor or a natural flavor, a coloring agent and a thickening agent (cheese, chocolate, or the like), pectic acid and a salt thereof, alginate and a salt thereof, an organic acid, a protective colloidal thickener, a pH adjusting agent, a stabilizer, a preservative, glycerin, alcohol, a carbonating agent used for carbonated beverages, and the like. These ingredients may be used alone or in combination, and proportions of these ingredients may also be appropriately selected by those skilled in the art.

Hereinafter, the present invention will be described in detail with reference to Examples.

However, the following Examples illustrate only the present invention in detail, and the present invention is not limited by the following Examples.

### [Example 1] Preparation of peptide

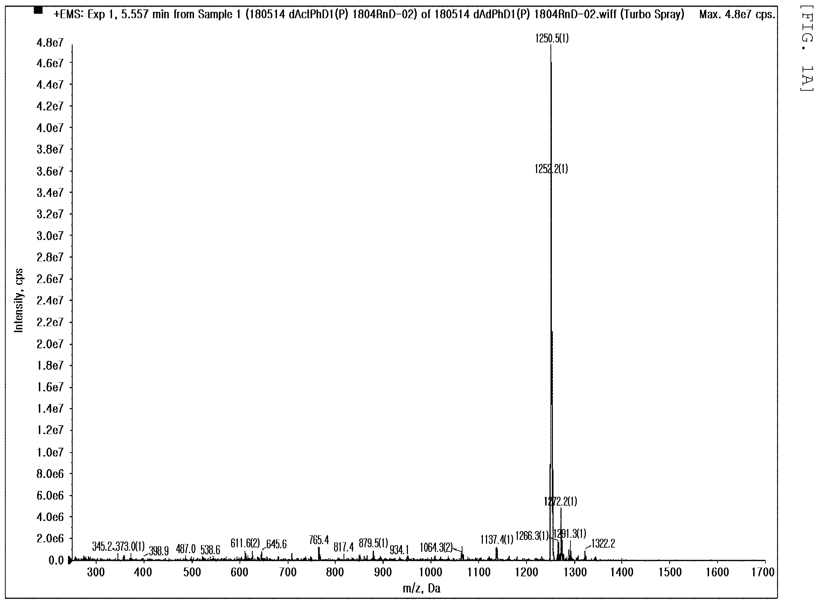

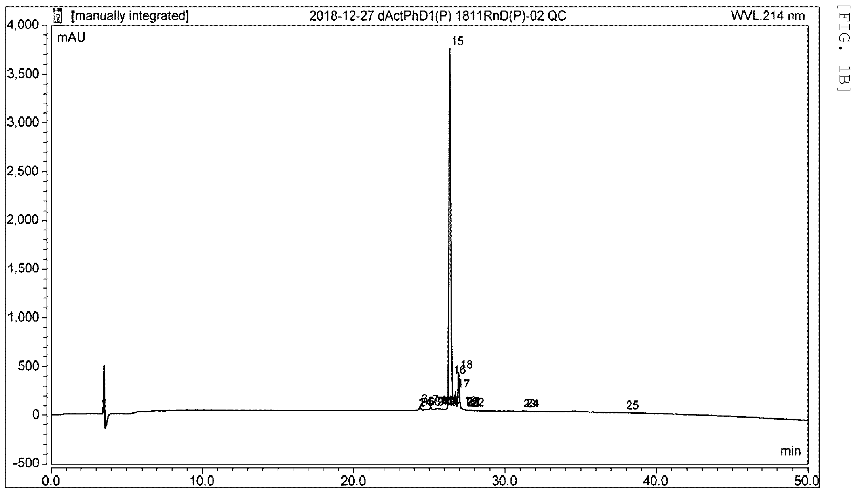

Peptides containing an amino acid sequence of SEQ ID NO: 1 described in Table 1 were synthesized by using an automatic peptide synthesizer (Milligen 9050, Millipore, USA), and these synthesized peptides were isolated and purified using C18 reverse-phase high-performance liquid chromatography (HPLC) (Waters Associates, USA). As a result of molecular weight analysis using 3200 QTRAP LC-MS/MS system, it was confirmed that the peptide of the present invention having a molecular weight of 1250.5 Da was synthesized (FIGS. 1A and 1B).

**[Table 1]**

| SEQ ID NO: | Peptide sequence |
|---|---|
| 1 | ACDWGLWWLC |

### [Experimental Example 1] Confirmation of promotion of ECM production in chondrocytes according to peptide treatment

In order to confirm the cartilage regeneration effect of treatment with the peptide of the present invention containing the amino acid sequence of SEQ ID NO: 1, human-derived chondrocytes were treated with the peptide of SEQ ID NO: 1, and then it was confirmed whether there was an increase in formation of glycosaminoglycan, which is an ECM component.

To this end, human C28/I2 chondrocyte cell line was distributed in a 96-well plate at a density of 3 × 10³ cells/well and cultured in DMEM medium (cat. 11995-065, Gibco) for 24 hours. After replacing the medium with a new medium, the peptide of SEQ ID NO: 1 was treated at concentrations of 30, 50, 100, and 150 ug/ml. The peptide of SEQ ID NO: 1 was treated at each concentration while changing the medium once every three days. After removing the medium on day 6, 60 µl of 3.7% formalin was added to the 96-well plate to be stained and fixed for 1 minute. After removing the 3.7% formalin, 70 µl of an alcian blue staining solution (50 ml of 3% acetic acid + 0.5 g of 1% Asian blue 8GX, pH 2.5) was added, and incubation was performed at 37°C for 24 hours. Thereafter, the staining solution was removed, washing with triple distilled water and drying were performed, and then observation was performed with a microscope.

As a result, it was confirmed that the peptide containing the amino acid sequence of SEQ ID NO: 1 of the present invention promoted ECM production in chondrocytes (see FIG. 2).

### [Experimental Example 2] Confirmation of promotion of ECM gene expression in chondrocytes according to peptide treatment

Human-derived chondrocytes were treated with the peptide containing the amino acid sequence of SEQ ID NO: 1, and then it was confirmed whether there was an increase in mRNA expression of collagen type II (COL2A1), aggrecan (ACAN), and proteoglycan core protein (PCP), which are ECM components, and GAPDH gene, which is a control.

To this end, human C28/I2 chondrocyte cell line was distributed in a 6-well plate at a density of 8.9 × 10⁴ cells/well and cultured in DMEM medium (cat. 11995-065, Gibco) for 24 hours. After replacing the medium with a new medium, the peptide of SEQ ID NO: 1 was treated at concentrations of 50 and 100 ug/ml. The peptide of SEQ ID NO: 1 was treated at each concentration while changing the medium once every three days. After removing the medium on day 9, cells were harvested and RNA was isolated. After synthesizing cDNA using a cDNA synthesis kit (Intron, Korea), PCR was performed for collagen type II, ACAN, PCP, and GAPDH genes using PCR pre-mix (Intron, Korea). Primers specific for the collagen type II, ACAN, PCP, and GAPDH are shown in Table 2.

**[Table 2]**

| Gene | Primer sequence |
|---|---|
| hCOL2A1 | F: CTATCTGGACGAAGCAGCTGGCA (SEQ ID NO: 2) |
| | R: ATGGGTGCAATGTCAATGATGG (SEQ ID NO: 3) |
| hACAN | F: GCAGGCTATGAGCAGTGTGA (SEQ ID NO: 4) |
| | R: GCATACCTCGGAAGCAGAAG (SEQ ID NO: 5) |
| hPCP | F: TGAGTCCTCAAGCCTCCTGT (SEQ ID NO: 6) |
| | R: GTGCCAGATCATCACCACAC (SEQ ID NO: 7) |
| GAPDH | F: ACCACAGTCCATGCCATCAC (SEQ ID NO: 8) |
| | R: TCCACCACCCTGTTGCTGTA (SEQ ID NO: 9) |

As a result, it was confirmed that the peptide containing the amino acid sequence of SEQ ID NO: 1 of the present invention promoted the expression of collagen type II, ACAN, and PCP genes associated with ECM production in chondrocytes (see FIG. 3).

### [Experimental Example 3] Confirmation of promotion of ECM production in three-dimensional culture of chondrocytes according to peptide treatment

Human-derived chondrocytes were treated with the peptide containing the amino acid sequence of SEQ ID NO: 1 in three-dimensional culture, and then it was confirmed whether there was an increase in the formation of glycosaminoglycan, which is an ECM component.

To this end, 3 × 10⁶ cells of human C28/I2 chondrocyte cell line were suspended in 1 ml of an alginate solution (1.25% alginate, 20 mM HEPES, 150 mM Nacl, pH 7.4), and the alginate solution containing the cells were added dropwise to a polymerization solution (100 mM CaCl₂, 10 mM HEPES, pH 7.4), thereby preparing beads. The prepared beads were washed twice with DPBS, and then the beads were distributed in a conical tube containing DMEM medium (5% FBS). The peptide of SEQ ID NO: 1 was treated at concentrations of 100 and 150 µg/ml, and the peptide of SEQ ID NO: 1 was treated at each concentration while changing the medium once every three days. After removing the medium on day 9, 3.7% formaldehyde was added and fixed for 6 hours. Dehydration was performed using a tissue processor (EtOH 70%, 80%, 90%, 100% I, II, xylene I, II in this order for 30 minutes each), and a paraffin block was prepared by embedding. The paraffin block was sectioned on a glass slide to a thickness of 0.4 µm and then completely dried for 24 hours or longer. The sections were hydrated (Xylene I, II, EtOH 100% I, II, 90%, 80%, 70% in this order for 3 minutes each) and washed three times by being immersed in running water for 10 seconds. After staining with alcian blue or toluidine blue for 1 hour, the sections were washed three times by being immersed in running water for 10 seconds. The sections were immersed in EtOH 90%, 100% I, II for 10 seconds, immersed in xylene I, II for 3 minutes each, and then mounted. The sections were dried for 24 hours or longer and then observed with a microscope.

As a result, it was confirmed that the peptide containing the amino acid sequence of SEQ ID NO: 1 of the present invention promoted the formation of glycosaminoglycan, which is an ECM component, in three-dimensional culture of chondrocytes (see FIG. 4).

### [Experimental Example 4] Confirmation of promotion of differentiation of mesenchymal stem cells into chondrocytes and ECM production according to peptide treatment

Adipose-derived mesenchymal stem cells were treated with the peptide containing the amino acid sequence of SEQ ID NO: 1, and then the effect on chondrogenic differentiation of the stem cells and ECM production was confirmed.

To this end, human adipose-derived mesenchymal stem cells (AD-MSCs) were distributed in a 96-well plate at a density of 1.5 × 10³ cells/well and cultured in DMEM medium (cat. 11995-065, Gibco) for 24 hours. After replacing the medium with DMEM medium containing 5% FBS, the peptide of SEQ ID NO: 1 was treated at concentrations of 30, 50, 100, and 150 ug/ml. The peptide of SEQ ID NO: 1 was treated at each concentration while changing the medium once every three days. After removing the medium on day 15, 60 µl of 3.7% formalin was added to the 96-well plate to be stained and fixed for 1 minute. After removing the 3.7% formalin, 70 µl of an alcian blue staining solution (50 ml of 3% acetic acid + 0.5 g of 1% Asian blue 8GX, pH 2.5) was added, and incubation was performed at 37°C for 24 hours. Thereafter, the staining solution was removed, washing with triple distilled water and drying were performed, and then observation was performed with a microscope.

As a result, it was confirmed that the peptide containing the amino acid sequence of SEQ ID NO: 1 of the present invention promoted the chondrogenic differentiation of the adipose-derived mesenchymal stem cells and the production of glycosaminoglycan, which is an ECM component (see FIG. 5).

### [Experimental Example 5] Confirmation of expression of ECM regulators in mesenchymal stem cells according to peptide treatment

Adipose-derived mesenchymal stem cells were treated with the peptide containing the amino acid sequence of SEQ ID NO: 1, and then it was confirmed whether there was an increase in expression of SOX9 protein, which is an ECM regulator.

To this end, human AD-MSC cells were distributed in a 96-well plate at a density of 1.5 × 10³ cells/well and cultured in DMEM medium (cat. 11995-065, Gibco) containing 10% FBS for 24 hours. After replacing the medium with DMEM medium containing 5% FBS, the peptide of SEQ ID NO: 1 was treated at concentrations of 50 and 100 ug/ml. The peptide of SEQ ID NO: 1 was treated at each concentration while changing the medium once every three days. At this time, as a positive control (CM), a solution containing dexamethasone (100 nM) + ascorbic acid (50 µM) + proline (40 µM) + TGFβ1 (10 ng/ml) + 1X ITS was used. After removing the medium on days 3, 7, and 14, cells were harvested, a cell lysate was prepared, and Western blot was performed using antibodies (Millipore, USA) against the SOX9 and SOX5 proteins.

As a result, it was confirmed that the peptide containing the amino acid sequence of SEQ ID NO: 1 of the present invention increased production of SOX9 protein, which is an ECM regulator, in adipose-derived mesenchymal stem cells (see FIGS. 6A to 6C).

### [Experimental Example 6] Confirmation of promotion of ECM gene expression in mesenchymal stem cells according to peptide treatment

Adipose-derived mesenchymal stem cells were treated with the peptide containing the amino acid sequence of SEQ ID NO: 1, and then it was confirmed whether there was an increase in expression of cartilage oligomeric matrix protein (COMP), collagen type 11A (COL 11A), PCP, and ACAN (aggrecan) genes associated with the ECM production.

To this end, human AD-MSC cells were distributed in a 96-well plate at a density of 1.5 × 10³ cells/well and cultured in DMEM medium (cat. 11995-065, Gibco) containing 10% FBS for 24 hours. After replacing the medium with DMEM medium containing 5% FBS, the peptide of SEQ ID NO: 1 was treated at concentrations of 50 and 100 ug/ml. The peptide of SEQ ID NO: 1 was treated at each concentration while changing the medium once every three days. After removing the me^{d}ium on days 3, 7, and 11, cells were harvested and RNA was isolated. After synthesizing cDNA using a cDNA synthesis kit (Intron, Korea), PCR was performed for COMP, COL 11A, PCP, ACAN, and GAPDH genes using PCR pre-mix (Intron, Korea). Primers specific for the COMP, COL 11A, PCP, ACAN, and GAPDH are shown in Table 3.

**[Table 3]**

| Gene | Primer sequence |
|---|---|
| hCOMP | F: AACTCAGGGCAGGAGGATGT (SEQ ID NO: 10) |
| | R: TGTCCTTTTGGTCGTCGTTC (SEQ ID NO: 11) |
| hCOL 11A | F: CCAGCCCGAACTTGTAAAGA (SEQ ID NO: 12) |
| | R: TGGACGCACAACCATCATAC (SEQ ID NO: 13) |
| hPCP | F: TGAGTCCTCAAGCCTCCTGT (SEQ ID NO: 6) |
| | R: GTGCCAGATCATCACCACAC (SEQ ID NO: 7) |
| hACAN | F: GCAGGCTATGAGCAGTGTGA (SEQ ID NO: 4) |
| | R: GCATACCTCGGAAGCAGAAG (SEQ ID NO: 5) |
| GAPDH | F: ACCACAGTCCATGCCATCAC (SEQ ID NO: 8) |
| | R: TCCACCACCCTGTTGCTGTA (SEQ ID NO: 9) |

As a result, it was confirmed that the peptide containing the amino acid sequence of SEQ ID NO: 1 promoted the expression of COMP, COL 11A, PCP, and ACAN genes associated with ECM production in the adipose-derived mesenchymal stem cells, (see FIGS. 7A to 7C).

Considering the results of Experimental Examples 1 to 6 as described above, it can be confirmed that the peptide containing the amino acid sequence of SEQ ID NO: 1 of the present invention promotes ECM synthesis, increases the expression of genes associated with the ECM production and ECM regulator genes, and promotes differentiation of mesenchymal stem cells into chondrocytes, by increasing the activity of chondrocytes, and has an excellent cartilage regeneration effect by increasing the expression of genes associated with ECM production and ECM regulator genes in mesenchymal stem cells. In addition, when the peptide of the present invention is treated in a larger amount, the above effect is exhibited to a greater extent, and thus, it can be seen that the cartilage regeneration effect seen in the results of the above experimental examples is due to the peptide of the present invention.

### [Experimental Example 7] Confirmation of expression level of inflammatory cytokine mRNA in cells induced by inflammation with TNFα

In addition to the cartilage regenerative activity confirmed in Experimental Examples 1 to 6, in order to confirm the anti-inflammatory effect according to the treatment with the peptide containing the amino acid sequence of SEQ ID NO: 1 of the present invention, mouse RAW264.7 macrophage cell line was treated with TNFα to induce inflammation, and the expression levels of TNFα and IL-1β, which are inflammatory cytokines, and Cox2 gene, which is an inflammation-inducing marker, were confirmed. The cytokine TNFα was treated to induce inflammation. Since TNFα corresponds to a signal transduction protein related to an inflammatory response, IL-1β corresponds to a cytokine that promotes an inflammatory response, and Cox2 is an inflammation-related protein, a degree of the inflammatory response may be confirmed by measuring the expression levels of TNFα, Cox2, and IL-1β genes.

To this end, mouse RAW264.7 cell line was distributed in a 6-well plate at a density of 2 × 10⁵ cells/well and cultured in α-MEM medium containing 10% FBS for 24 hours. The medium was replaced with serum-free α-MEM medium (1% penicillin) and then fasted for 6 hours. The peptide of SEQ ID NO: 1 was treated at concentrations of 100 and 150 µg/ml, and then incubation was performed for 1 hour. After 1 hour, the cells were treated with 20 nM TNF-α and incubated for 24 hours. Thereafter, the cells were harvested and RNA was isolated. After synthesizing cDNA using a cDNA synthesis kit (Intron, Korea), PCR was performed for TNFα, IL-1β, Cox2, and GAPDH genes using PCR pre-mix (Intron, Korea). Primers specific for the TNFα, IL-1β, Cox2, and GAPDH genes are shown in Table 4.

**[Table 4]**

| Gene | Primer sequence |
|---|---|
| TNFα | F: CGT CAG CCG ATT TGC TAT CT (SEQ ID NO: 14) |
| | R: CGG ACT CCG CAA AGT CTA AG (SEQ ID NO: 15) |
| IL-1β | F: CAA GGA GAA CCA AGC AAC GA (SEQ ID NO: 16) |
| | R: TTG GCC GAG GAC TAA GGA GT (SEQ ID NO: 17) |
| Cox2 | F: TGA GTG GTA GCC AGC AAA GC (SEQ ID NO: 18) |
| | R: CTG CAG TCC AGG TTC AAT GG (SEQ ID NO: 19) |
| GAPDH | F: ACCACAGTCCATGCCATCAC (SEQ ID NO: 8) |
| | R: TCCACCACCCTGTTGCTGTA (SEQ ID NO: 9) |

As a result, it was confirmed that the peptide containing the amino acid sequence of SEQ ID NO: 1 of the present invention inhibited the expression of inflammatory cytokine genes induced by the treatment of inflammatory cytokines in mouse macrophages (see FIG. 8).

### [Experimental Example 8] Confirmation of expression level of inflammatory cytokine mRNA in cells induced by inflammation with TNFα

In addition to the results of Experimental Example 7, in order to further confirm the anti-inflammatory effect of the treatment with the peptide containing the amino acid sequence of SEQ ID NO: 1 of the present invention, mouse RAW264.7 macrophage cell line was treated with TNFα to induce inflammation, and the expression level of COX2 protein, which is an inflammation-inducing marker, was confirmed.

To this end, mouse RAW264.7 cell line was distributed in a 6-well plate at a density of 2 × 10⁵ cells/well and cultured in α-MEM medium containing 10% FBS for 24 hours. The medium was replaced with serum-free α-MEM medium (1% penicillin) and then fasted for 6 hours. The peptide of SEQ ID NO: 1 was treated at concentrations of 100 and 150 µg/ml, and then incubation was performed for 1 hour. After 1 hour, the cells were treated with 20 nM TNF-α and incubated for 24 hours. Thereafter, the cells were harvested, a cell lysate was prepared, and then Western blot was performed using antibodies against the COX2 protein (Cell Signaling Technology, USA).

As a result, it was confirmed that the peptide containing the amino acid sequence of SEQ ID NO: 1 of the present invention inhibited the increase in inflammatory proteins caused by the treatment of inflammatory cytokines in mouse macrophages (see FIG. 9).

Considering the results of Experimental Examples 7 and 8 as described above, when cells in which an inflammatory response is induced are treated with the peptide containing the amino acid sequence of SEQ ID NO: 1 of the present invention, the amount of protein or expression levels of genes related to the inflammatory response is reduced, and thus, it can be confirmed that the peptide containing the amino acid sequence of SEQ ID NO: 1 of the present invention has an inhibitory effect on the inflammatory response. In addition, when the peptide of the present invention is treated in a larger amount, the above effect is exhibited to a greater extent, and thus, it can be seen that the inhibitory effect of the inflammatory response seen in the results of the above experimental examples is due to the peptide of the present invention.

### [Experimental Example 9] Confirmation of inhibitory effect on osteoclast differentiation induced by RANKL

In addition to the cartilage regenerative activity confirmed in Experimental Examples 1 to 6 and the anti-inflammatory effect confirmed in Experimental Examples 7 and 8, the effect of inhibiting osteoclast differentiation according to the treatment with the peptide containing the amino acid sequence of SEQ ID NO: 1 of the present invention was confirmed.

### <9-1> Confirmation of osteoclast differentiation inhibitory effect of peptide of the present invention

Mouse RAW264.7 cell line was distributed in a 96-well plate at a density of 1.7 × 10³ cells/well and cultured in DMEM medium for 24 hours. After 24 hours, the peptide of SEQ ID NO: 1 was treated at concentrations of 10, 30, 30, 100, and 150 µg/ml together with RANKL, and then differentiation of osteoclasts was induced for 4 days. After removing the medium, the cells were fixed for 30 seconds and washed twice with double distilled water. Staining was performed with 100 ul of a TRAP staining solution (acid phosphatase kit, Sigma aldrich) at 37°C for 30 minutes. Thereafter, the staining solution was removed, washing with double distilled water three times and drying were performed, and then observation was performed with a microscope.

As a result, it was confirmed that the peptide containing the amino acid sequence of SEQ ID NO: 1 of the present invention inhibited the differentiation into osteoclasts induced by RANKL in mouse macrophages (see FIG. 10A) .

### <9-2> Confirmation of cytotoxicity of peptide of the present invention

The effect of the peptide of the present invention on cell proliferation was confirmed using CCK-8 assay. CCK-8 reagent is reduced by cellular dehydrogenase to produce a colored product, and the proliferation of cells may be confirmed through a difference in absorbance using this.

To this end, mouse RAW264.7 cell line was distributed in a 96-well plate at a density of 1.7 × 10³ cells/well and cultured in DMEM medium for 24 hours. After 24 hours, the peptide of SEQ ID NO: 1 was treated at concentrations of 10, 30, 50, 100, and 150 µg/ml, and then incubation was performed for 3 days. After removing the medium, CCK-8 solution (Dojindo, CCK-8 kit) was diluted 10 times in the culture medium and treated at 100 µl each. During incubation at 37°C, absorbance at a wavelength of 450 nm was measured using a microplate reader at 30-minute intervals.

As a result, when treated with the peptide containing the amino acid sequence of SEQ ID NO: 1 of the present invention, rather, the absorbance was higher, and thus it was confirmed that the peptide of the present invention was not toxic to mouse macrophages (see FIG. 10B).

### <9-3> Confirmation of inhibitory effect of peptide of the present invention on TRAP activity related to osteoclast differentiation

Mouse RAW264.7 cell line was distributed in a 96-well plate at a density of 1.7 × 10³ cells/well and cultured in DMEM medium for 24 hours. After 24 hours, the peptide of SEQ ID NO: 1 was treated at concentrations of 10, 30, 50, 100, and 150 µg/ml together with RANKL, and then differentiation of osteoclasts was induced for 4 days. The cells were treated with 100 ul of a TRAP activity solution (TRAP buffer (0.1 sodium citrate + 15 ml of 50 µM sodium tartrate [pH 5.0]) + 4-nitrophenly phosphate disodium salt hexahydrate, sigma, 1 tablet mix), incubated at 37°C for 1 hour, and treated with 10 µl of 2 N NaOH. Absorbance at a wavelength of 405 nm was measured using a plate reader.

As a result, it was confirmed that the peptide containing the amino acid sequence of SEQ ID NO: 1 of the present invention inhibited the activity of TRAP, which is a marker of osteoclasts, in mouse macrophages in a concentration-dependent manner (see FIG. 10C).

### [Experimental Example 10] Confirmation of inhibitory effect on osteoclast differentiation induced by RANKL

Mouse RAW264.7 cell line was distributed in a 96-well plate at a density of 1.7 × 10³ cells/well and cultured in DMEM medium for 24 hours. After 24 hours, the peptide of SEQ ID NO: 1 was treated at concentrations of 50 µg/ml and 100 ug/ml together with RANKL, and then differentiation of osteoclasts was induced for 4 days. After removing the medium on day 1 of induction, cells were harvested, a cell lysate was prepared, and Western blot was performed using antibodies (Millipore, USA) against NFATc1 protein.

As a result, it was confirmed that the peptide containing the amino acid sequence of SEQ ID NO: 1 of the present invention inhibited the production of NFATc1 protein, which is a main transcription factor in the differentiation process of adipose-derived mesenchymal stem cells into osteoclasts (see FIG. 11).

### [Experimental Example 11] Confirmation of inhibition of actin ring formation in osteoclasts induced by RANKL

In addition to the results of Experimental Examples 9 and 10, in order to further confirm the effect of inhibiting osteoclast differentiation according to the treatment with the peptide containing the amino acid sequence of SEQ ID NO: 1 of the present invention, mouse RAW264.7 macrophage cell line was treated with RANKL to induce differentiation into osteoclasts, and it was confirmed whether the peptide of the present invention inhibited the formation of actin rings, which are essential for osteoclast differentiation.

To this end, mouse RAW264.7 cell line was distributed in a 96-well plate at a density of 5 × 10³ cells/well and cultured in DMEM medium for 24 hours. After 24 hours, the peptide of SEQ ID NO: 1 was treated at concentrations of 50 and 100 ug/ml together with RANKL, and then differentiation of osteoclasts was induced for 4 days. After removing the medium, the cells were washed three times with cooled PBS and fixed with 4% paraformaldehyde for 30 minutes. The cells were washed three times with cooled PBS for 5 minutes, and then the cells were permeabilized with 0.3% Triton X-100 in PBS for 30 minutes. The cells were washed three times with cooled PBS for 5 minutes, and the cells were blocked with filtered 3% BSA in PBS for 1 hour with stirring. Incubation was performed with primary antibodies (Rhodamin (Red) [Invitrogen^{™}]) diluted to 1:100 with a blocking medium with stirring for 2 hours. The cells were blocked from light and washed three times with PBS for 15 minutes with stirring, DAPI mounting medium (VECTASHIELD^{®} MOUNTING MEDIUM with DAPI) was added, and then each well was covered with a cover slip and observed with a microscope.

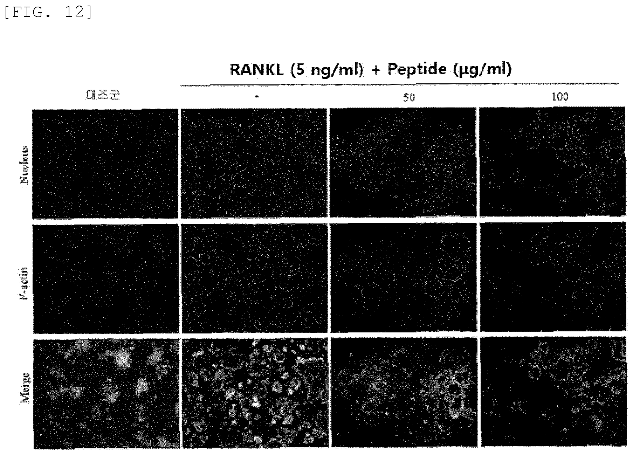

As a result, it was confirmed that the peptide containing the amino acid sequence of SEQ ID NO: 1 of the present invention inhibited the formation of actin rings, which are essential for differentiation of mouse macrophages into osteoclasts (see FIG. 12).

Considering the results of Experimental Examples 9 to 11 as described above, it can be confirmed that the peptide containing the amino acid sequence of SEQ ID NO: 1 of the present invention may inhibit differentiation into osteoclasts, and may be applied to the prevention or treatment of osteoporosis. In addition, when the peptide of the present invention is treated in a larger amount, the above effect is exhibited to a greater extent, and thus, it can be seen that the effect of inhibiting osteoclast differentiation seen in the results of the above experimental examples is due to the peptide of the present invention.

### [Preparation Example 1] Preparation of pharmaceutical composition

### <1-1> Preparation of powder

2 g of peptide of Example 1
1 g of lactose

The above ingredients were mixed and filled into an airtight bag to prepare powder.

### <1-2> Preparation of tablets

100 mg of peptide of Example 1
100 mg of corn starch
100 mg of lactose
2 mg of magnesium stearate

The above ingredients were mixed and then tableted according to a conventional tablet preparation method, thereby preparing tablets.

### <1-3> Preparation of capsules

100 mg of peptide of Example 1
100 mg of corn starch
100 mg of lactose
2 mg of magnesium stearate

The above ingredients were mixed and then filled in gelatin capsules according to a conventional capsule preparation method, thereby preparing capsules.

### <1-4> Preparation of pills

1 g of peptide of Example 1
1.5 g of lactose
1 g of glycerin
0.5 g of xylitol

The above ingredients were mixed and then pills were prepared to weight 4 g per pill according to a conventional method.

### <1-5> Preparation of granules

150 mg of peptide of Example 1
50 mg of soybean extract
200 mg of dextrose
600 mg of starch

The above ingredients were mixed, 100 mg of 30% ethanol was added and dried at 60°C to form granules, and then the granules were filled in a bag.

### [Preparation Example 2] Preparation of health functional food composition

### <2-1> Preparation of flour foods

0.5 to 5.0 parts by weight of the peptide of Example 1 was added to flour, and bread, cakes, cookies, crackers, and noodles were prepared using the mixture.

### <2-2> Preparation of soups and gravies

0.1 to 5.0 parts by weight of the peptide of Example 1 was added to soups and gravies to prepare health-enhancing meat products and noodle soups and gravies.

### <2-3> Preparation of dairy products

5 to 10 parts by weight of the peptide of Example 1 was added to milk, and various dairy products such as butter and ice cream were prepared using the milk.

### <2-4> Preparation of powdered foods

Brown rice, barley, glutinous rice, and coix seeds were pre-gelatinized and dried using a known method, and the pre-gelatinized and dried product was roasted and then prepared into powder having a particle size of 60 mesh using a grinder. Black beans, black sesame seeds, and perilla seeds were also steamed and dried using a known method, and the steamed and dried product was roasted and then prepared into powder having a particle size of 60 mesh using a grinder. The grains and seeds prepared above and the peptide of Example 1 were mixed at the following ratio.

Grains (30 parts by weight of brown rice, 15 parts by weight of coix seeds, and 20 parts by weight of barley), seeds (7 parts by weight of perilla seeds, 8 parts by weight of black beans, and 7 parts by weight of black sesame seeds), peptide of Example 1 (3 parts by weight), Reishi mushroom (0.5 parts by weight), and rehmannia (0.5 parts by weight)

### <2-5> Preparation of healthy drinks

5 g of the peptide of Example 1 and auxiliary ingredients such as high fructose corn syrup (0.5%), oligosaccharide (2%), sugar (2%), table salt (0.5%), and water (75%) were mixed homogeneously and instantaneously sterilized, and the mixture was packaged in small containers such as a glass bottle and a plastic bottle to prepare healthy drinks.

As described above, although the present invention has been described in detail with reference to only embodiments described herein, it will be apparent to those skilled in the art that various modifications and alterations may be made without departing from the technical idea of the present invention, and it is obvious that these modifications and alterations are within the following claims.

## Claims

1. A peptide comprising an amino acid sequence of SEQ ID NO: 1.

2. The peptide of claim 1, wherein the peptide has one or more physiological activities selected from the group consisting of cartilage regenerative activity, anti-inflammatory activity, and osteoporosis inhibitory activity.

3. The peptide of claim 1, wherein the peptide promotes differentiation of stem cells into chondrocytes.

4. The peptide of claim 3, wherein the stem cells are one or more selected from the group consisting of cord blood-derived stem cells, peripheral blood-derived stem cells, bone marrow-derived stem cells, and mesenchymal stem cells.

5. The peptide of claim 1, wherein the peptide increases synthesis of an extracellular matrix (ECM) in chondrocytes.

6. The peptide of claim 1, wherein the peptide increases expression of one or more genes selected from the group consisting of collagen type II (COL2A1), aggrecan (ACAN), and proteoglycan core protein (PCP) in chondrocytes.

7. The peptide of claim 1, wherein the peptide inhibits expression of inflammatory cytokines.

8. The peptide of claim 7, wherein the inflammatory cytokines are one or more selected from the group consisting of TNFα, IL-1β, COX2, IL-6, IL-17, and IFNγ.

9. The peptide of claim 1, wherein the peptide inhibits differentiation of macrophages into osteoclasts.

10. A pharmaceutical composition for regenerating cartilage, comprising the peptide of any one of claims 1 to 9 as an active ingredient.

11. A pharmaceutical composition for preventing or treating an inflammatory disease, comprising the peptide of any one of claims 1 to 9 as an active ingredient.

12. A pharmaceutical composition for preventing or treating osteoporosis, comprising the peptide of any one of claims 1 to 9 as an active ingredient.

13. A health functional food for regenerating cartilage, comprising the peptide of any one of claims 1 to 9 as an active ingredient.

14. A health functional food for preventing or ameliorating an inflammatory disease, comprising the peptide of any one of claims 1 to 9 as an active ingredient.

15. A health functional food for preventing or ameliorating osteoporosis, comprising the peptide of any one of claims 1 to 9 as an active ingredient.
